Europäisches Patentamt

European Patent Office

Office européen des brevets

(19)

(11) Publication number: **0 318 776**
**A1**

# (12) EUROPEAN PATENT APPLICATION

(21) Application number: **88119232.2**

(22) Date of filing: **18.11.88**

(51) Int. Cl.4: **A61N 1/30**

(30) Priority: **04.12.87 US 128789**

(43) Date of publication of application:
**07.06.89 Bulletin 89/23**

(84) Designated Contracting States:
**AT BE CH DE ES FR GB GR IT LI LU NL SE**

(71) Applicant: **Tapper, Robert**
**1935 Armacost Avenue**
**Los Angeles California 90025(US)**

(72) Inventor: **Tapper, Robert**
**1935 Armacost Avenue**
**Los Angeles California 90025(US)**
Inventor: **James, Carter A.**
**20437 Acre Street**
**Canoga Park California 91306(US)**
Inventor: **Baker, Bernard R.**
**5244 James Avenue**
**Oakland California 94618(US)**

(74) Representative: **Baillie, Iain Cameron et al**
**c/o Ladas & Parry Isartorplatz 5**
**D-8000 München 2(DE)**

(54) Method and apparatus for minimizing skin injury with electrode use.

(57) A method and apparatus for minimizing skin irritation and iontophoretic burns with electrode (10) use by interposing, within an electrical circuit, an ion-exchange material (18) or a sacrificial electrode between an electrical power source (17) and a patient's skin, to remove caustic or acidic ions electrolytically generated by the application of a current through the circuit. The ion-exchange material (18) may include an ion-exchange resin, retained within a housing (12) or an electroconductive gel matrix. The sacrificial electrode is formed of a corrosive metal which oxides or reduces the acidic or caustic ions. Various electrode (10) structures incorporating the ion-exchange material (18) and/or the sacrificial electrode are disclosed.

EP 0 318 776 A1

# METHOD AND APPARATUS FOR MINIMIZING SKIN INJURY WITH ELECTRODE USE

Technical Field

This invention relates generally to a method and apparatus for minimizing or eliminating skin irritation and burns commonly encountered with electrical therapies, such as iontophoretic treatment or the like, and, more particularly, to a method and apparatus which interposes an ion-exchange medium or a sacrificial electrode for removing caustic or acidic ions from solution, the common cause of such irritation and burns.

Background of the Invention

A wide variety of electrodes has been used for galvanic or iontophoretic treatment. Medical galvanism is the administration of "straight" galvanic current without introducing drugs into the skin of the patient. Straight galvanic current can be described as a predominantly unidirectional direct current flowing between two bioelectrodes. This includes pulsating direct current, so long as there is a predominantly unidirectional flow. Iontophoresis refers to the use of electrical means for infusing a medication through an intact membrane. As with medical galvanism, iontophoresis requires the administration of a substantially straight galvanic current to the patient's skin.

One of the principle problems with the prior art devices has been tissue burns where the bioelectrode is connected to the patient. Once the skin or tissue is burned, the resistance at the site of the burn decreases, allowing for an additional increase in current flow and thus ion migration at that site. This compounds the danger of serious burns to skin or other tissue. If a cut exists somewhere at the location of contact of the bioelectrode with the skin or tissue, then the ions tend to migrate toward and concentrate at the cut thereby, further increasing the danger of a burn.

Responsive to this problem, the general approach was to maximize the contact area of the bioelectrode, to diffuse the caustic or acidic chemicals applied to the skin over a larger surface area. However, smaller bioelectrodes would be easier to use and manipulate. For example, dental probes would require small, adjacent bioelectrodes for use within a patient's mouth.

The inventors have identified the burn and irritation causing phenomenon to be deleterious ions appearing beneath these bioelectrodes. More specifically, ions are electrolytically generated at the anode (positive output) and cathode (negative output) when in wet contact with the patient. Because of electrochemical laws and differences in the ionization ability of different chemical elements, hydroxide ions ($OH^-$) and hydrogen gas ($H_2$) appear at the negative electrode and hydrogen ($H^+$) or hydronium ($H_3O^+$) ions and oxygen gas ($O_2$) appear at the positive electrode.

Iontophoresis involves the infusion of a medication through the skin for therapeutic purposes by the application of an electric current between bioelectrodes attached to the patient's skin where the patient is the electrical load connected between the bioelectrodes. The solution containing the medical compound or ions is placed under a first bioelectrode having the same charge as the drug. Ions migrate from the solution through the skin due to the electric current applied through the solution and skin. A second or return bioelectrode, opposite in charge to the drug, is placed at a site so that current flows from one bioelectrode to the other. The operator then selects an electric current below the level of the patient's pain threshold and applies it through the medication in contact with the skin for an appropriate length of time. The electrical current applied across these bioelectrodes cause the ions to migrate into the skin. This technique has been discussed in a number of scientific journals (see Tyle, and Praveen, Pharmaceutical Research, 3:318, (1986), "Review of Iontophoretic devices for Drug Delivery"; and Sloan and Soltani, J. Am. Acad. Dermatol., 15: 671-684 (1986), "Iontophoresis in Dermatology"); and a number of prior art patents including U.S. Patent No. 4,416,274, issued November 22, 1983 to Jacobsen, et al. Examples of bioelectrodes which are generally useful for making electrical contact with the skin are described in U.S. Patent No. 3,862,633; 3,945,384 and 3,973,557.

Attempts to minimize iontophoretic burns are disclosed in U. S. Patent Nos. 4,164,226 and 4,211,222, issued to Tapper, et al, teaching the use of a thick wool intervener that physically absorbs the hydroxide ions. These pads are less effective, however, in stopping the hydrochloric acid coming from the positive electrode. In this regard, it is of particular importance that the injurious ions at the positive electrode be controlled, because a large majority of the medicinal compounds iontophoretically administered to patients are placed beneath the positive electrode.

Thus, a method or device which reduces the irritation of deleterious ions, e.g. hydronium or hydroxide ions, generated electrolytically adjacent to the electrodes is particularly desirable. In one response, the hydronium ions were neutralized by adding a baking soda solution (NaHCO$_3$). In U.S. Patent No. 4,416,274 issued to Jacobsen, et al, a phosphate buffer solution was used to reduce iontophoretic burns. However, buffers have undesirable side-effects, e.g. chemically interacting with the drug or competing with the medicine for infusion through the skin.

Hence, those concerned with the development and use of bioelectrodes, and more particularly iontophoretic bioelectrodes, have long recognized the need for an improved method and apparatus for enabling extended bioelectrode contact with the patient's skin during therapy without suffering from undesirable skin irritation or burns. The present invention clearly fulfills all of these needs.

Summary of the Invention

Briefly, and in general terms, the present invention provides a new and improved method and apparatus for reducing skin irritation and burns wherein novel methods and apparatus are employed for exchanging the deleterious skin-injuring ions for a more benign chemical species, enabling extended periods of bioelectrode contact with the patient's skin without suffering from the adverse side-effects of previously available bioelectrodes.

Basically, the present invention is directed to an improved bioelectrode administration method and apparatus which exchanges the hydronium or hydroxide ions for their more benign salt equivalents, e.g. free sodium ions (Na$^+$) or free chloride ions (Cl$^-$) in a bioelectrode environment, such as iontophoretic treatment and the like.

In a presently preferred embodiment of the invention, by way of example and not necessarily by way of limitation, an ion conversion material, e.g. an ion-exchange resin which exchanges hydroxide or hydronium ions for Cl$^-$ or Na$^+$), is interposed between an output electrode and the patient's skin. As a result, the acidic or caustic ions, e.g. H$^+$ or OH$^-$, are exchanged for other ions, e.g. Na$^+$ or Cl$^-$, thereby reducing skin irritation and burn.

An alternative method or apparatus for reducing skin injury, in accordance with the invention, involves the interposition of a corrosive metal between the metal electrode and the skin surface. The corrosive metal acts as a sacrificial electrode or chemical decoy to react with the irritation ions and oxidize or reduce them to less damaging compounds, e.g. metal oxides.

These and other objects and advantages of the invention will become apparent from the following more detailed description, when taken in conjunction with the accompany drawings of illustrative embodiments.

Description of the Drawings

Figure 1 is a plan view of bioelectrode device embodying features of the present invention and shown placed on a human subject's skin;

Figure 2 is an enlarged sectional view, taken substantially along the line 2-2 of the bioelectrode of Figure 1;

Figure 3 is an enlarged, sectional view of a modified embodiment of the bioelectrode of Figure 2;

Figure 4 is an enlarged, fragmentary sectional view of still another modified embodiment of the bioelectrode of Figure 2;

Figure 5 is an enlarged, fragmentary sectional view of a further embodiment of the present invention;

Figure 6 is an enlarged, fragmentary sectional view of a modified embodiment of the bioelectrode of Figure 1;

Figure 7 is an enlarged, sectional view of still a further embodiment of the present invention; and

Figure 8 is an enlarged, fragmentary sectional view of another embodiment of the present invention.

Detailed Description of the Invention

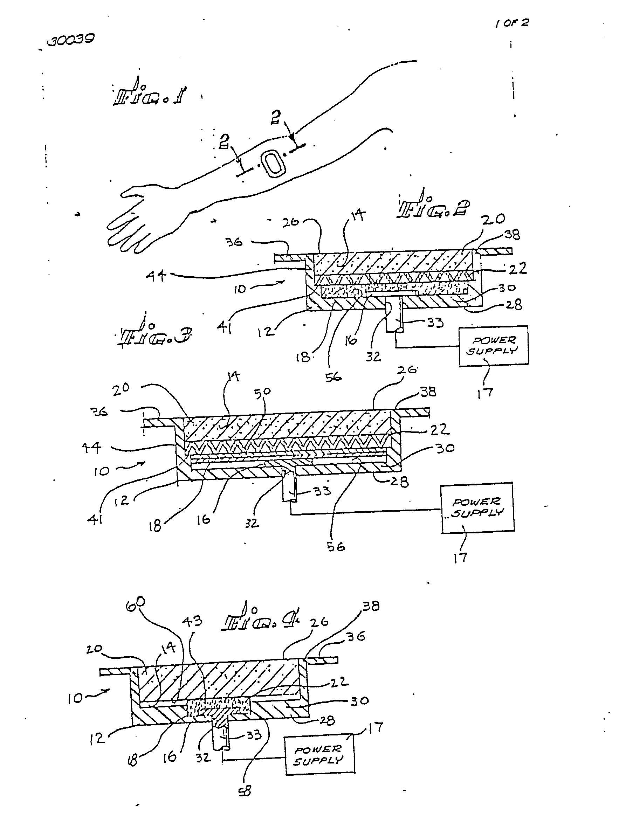

As shown in the exemplary drawings for the purposes of illustration, the present invention is principally concerned with methods and apparatus for reducing skin injury when using bioelectrodes, particularly in a medical galvanic or iontophoretic treatment environment and the like. A bioelectrode assembly in accordance with the invention and referred to generally by the reference numeral 10 in Figure 1, is shown in a

3

typical position upon a patient's arm and provided with a novel construction which results in decreased injury to the patient's skin to which it is attached. Of course, it will be appreciated that the bioelectrode may be located anywhere on the patient without in any way departing from the principle of the invention. Throughout the drawings, like reference numerals denote like corresponding structural elements.

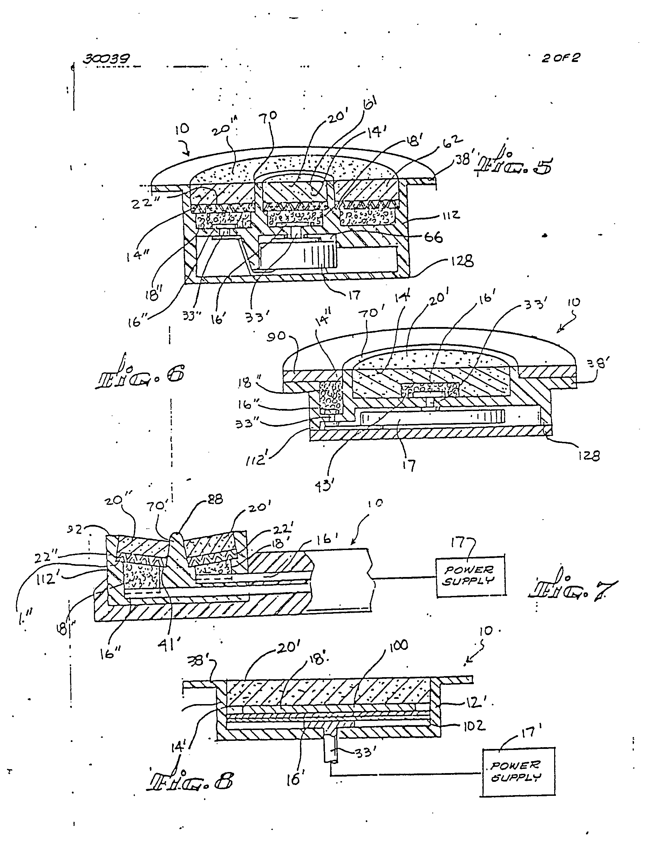

As best observed in Figures 2-4, the bioelectrode 10 includes a housing 12, defining at least one interior cavity 14. A metal electrode 16, disposed within the housing 12, is connected with an electrical power source 17 which is typically a regulated d.c. source. Ion conversion material 18, is disposed within the housing 12 and interposed between the metal electrode 16 and the patient's skin.

For iontophoretic applications, the bioelectrode 10 includes dispenser 20, for receiving and releasing the compound to be iontophoretically applied to the patient. Dispensers 20 may be any material suitable for receiving and releasing the iontophoretic compound, e.g. fibrous pads or gels. The dispenser 20 is in direct contact with the patient's skin and in the electrical circuit with the power source 17 for supplying an electrical current to infuse the compound into the patient's skin. A retainer 22, interposed between the dispenser 20 and the ion conversion material 18, retains the ion conversion material within the housing 12, between the metal electrode 16 and the patient's skin.

Consequently, the deleterious ions electrolytically generated adjacent to the metal electrode 16, are exchanged for less deleterious or injurious ions and bound to the ion conversion material 18, effectively removing the deleterious ions from solution. For purposes of illustration and not necessarily by way of limitation, "deleterious" or "injury causing" ions may be defined as the ionic constituents hydronium and hydroxide or their ionic equivalents, generated by the solubilizing of acids or bases, e.g. HCL or NaOH. The exchange of these deleterious ions for more benign or less irritating ions mitigates the irritation and burning of the patient's skin earlier described.

The improved bioelectrode 10 of the present invention provides a relatively inexpensive and simple method and apparatus for exchanging, reducing or oxidizing deleterious ions electrolytically generated beneath the respective bioelectrodes. For example, irritation or burns caused by the application of electric current to iontophoretic bioelectrodes 10 in contact with the patient's skin and greatly reduced. Iontophoretic bioelectrodes 10 are generally used in conjunction with saline solutions, e.g. body fluids or prepared solutions including dissolved sodium chloride. The ion conversion material 18 of the iontophoretic bioelectrode 10 reduces the free hydrogen ions ($H^+$ or hydronium ions ($H_3O^+$), oxidizes the hydroxide ion ($OH^-$), or exchanges them for $Na^+$ or $Cl^-$, in order to remove the injury causing ions from the solution.

More specifically, the housing 12 provides means for supporting and positioning various other elements of the iontophoretic electrode 10 structure. As shown in Figures 2-4, the housing 12 has an open patient-contacting end 26 for contacting the patient's skin and a closed back end 28, further removed from the patient's skin. The housing 12 is generally in the form of a cup having an interior cavity or well 14 defined therein. A bottom wall 30, adjacent the back end 28, substantially encloses the back end. An aperture 32 is formed within the bottom wall 30 to pass an electrical contacting means 33 therethrough. An annular flange 36 extends outward radially from a lip 38 of the housing 12 to facilitate the attachment of the bioelectrode 10 to the patient's skin.

As shown in Figures 2 and 3, the improved bioelectrode 10 may include an annular shelf 41 extending radially inward from a cup wall 44 to facilitate the attachment of the retainer 22 to the housing 12.

Referring to Figures 2-4, the power source 17, e.g. a battery or other means for generating a current, is electrically connected by electrical contacting means 33, e.g. a wire or strip of electroconductive material, to the metal electrode 16 mounted at the bottom wall 30 of the interior cavity 14. Depending upon whether the metal electrode 16 is electrically connected to the anode or cathode of the power source 17, the metal electrode 16 is formed of a material best suited for resisting the effects of the respective irritating ions generated adjacent thereto. By way of example, stainless steel is preferably used at the negative electrode (cathode) because of it's compatibility with NaOH. At the positive electrode (anode) platinum is preferred. However, for economy, aluminum or aluminum alloys, e.g. Aluminum 1100 sold by Kaiser Aluminum of Fontana, California, may be used.

Interposed between the metal electrode 16 and the patient's skin, within the interior cavity 14, is the ion conversion material 18. In one embodiment, as shown in Figure 2, the ion conversion material is an ion-exchange resin. The ion-exchange resin may be defined as any material which causes a reversible interchange of ions between a solid and liquid phase in which there is no permanent change in the structure of the solid, the solid being the ion-exchange resin. The ion-exchange resin is a solid organic polymer backbone with a multitude of ionic binding sites attached thereto. These organic sites bind to mobile ions, e.g. $Cl^-$, $OH^-$, $Na^+$, or $H^+$. Saline containing media or body fluids including sodium chloride (NaCl) are often in contact with iontophoretic bioelectrodes 10. Thus, ion-exchange resins binding with $Na^+$ and $Cl^-$ are particularly useful. While the particular ion-exchange resins typically employed in the practice of the invention exchange $Cl^-$ for $OH^-$ and $Na^+$ for $H^+$, other ion-exchange resins are contemplated for substitution in other applications. By way of example, the reactions involved are:

ANION EXCHANGE $R_aCl + OH^- = R_aOH + Cl^-$.
CATION EXCHANGE $R_cNa + H^+ = R_cH + Na^+$.

As a result, the anion exchange resin converts free hydroxide ions to free chloride ions by the reversible binding described above. The cation exchange resin converts free hydrogen ions (e.g. solubilized hydrochloric acid) to free $Na^+$ ions (e.g. solubilized sodium chloride). Thus deleterious ions would be converted to less irritating salt equivalents, minimizing skin irritation and burning.

As is widely known in the art, such ion-exchange resins can be regenerated and reused by soaking in at least five percent NaCl after treatment. After soaking in salt solution, the resin could be rinsed with de-ionized water to remove the regenerating salt. The resins typically come in the form of small spheres. The small spheres may very from 200/400 mesh (a fine powder) to 20/50 mesh (small granules).

It has been determined in the practice of the invention, that one cubic centimeter ("c.c.") of resin used in a one-inch diameter bioelectrode 10, similar to that in Figure 2, will last approximately one hundred and twenty milliampere-minutes (ma-mins) before allowing an iontophoretic burn (cathode), as compared to a control electrode with no resin, which will allow an iontophoretic burn in about six ma-mins. A milliampere-minute is defined as an amount of current expressed in milliamperes multiplied by the amount of time the current is applied in terms of minutes. A burn was defined as the appearance of a grayish/red spot on the skin at the bioelectrode site. The bioelectrode was periodically peeled from the skin to check for a burn. Doubling the volume of resin to about two c.c.'s increase the burn free treatment time to about one hundred and forty-five ma-mins (a twenty-one percent increase).

Initial experiments in practicing the invention indicate that the particular size mesh of the resin does not appear to have any substantial affect on the results. See Table 1 below:

TABLE 1

| TREATMENT TIME BEFORE BURN AFTER APPLYING 2.0 ma TO THE BIOELECTRODE | |
|---|---|
| Resin: size (amount) | |
| 200/400 mesh resin (1cc) | 58 minutes |
| 20/50 mesh resin (1cc) | 56 minutes |
| 200/400 mesh resin (2cc) | 70 minutes |
| 20/50 mesh resin (2cc) | 75 minutes |

In addition, the specific type of ion conversion material 18 used in the bioelectrode 10, depends upon the polarity of the metal electrode 16. For example, an anion exchange resin, e.g. AG1 - X8 200/400 mesh anion exchange resin sold under the tradename "DOWEX" by Dow Chemical Company of Midland, Michigan, is used adjacent the cathode, because of the increased ability to exchange with hydroxide ions. Likewise, cation exchange resin, e.g. 50 W X8 200/400 mesh and/or XUS - 400 9000 20/50 mesh (sodium form) ion-exchange resin sold under the tradename "DOWEX" by Dow Chemical is used adjacent the anode, i.e. positive polarity electrode. This facilitates the removal of the specific ions generated adjacent the respective electrode polarity.

In addition to causing damage to the skin, hydroxide and hydronium ions are also highly conductive, which interferes with treatment efficiency. During iontophoretic treatment, it is important to minimize these electrolytically generated, naturally occuring ions to reduce inhibiting competition with desirable drug ions for infusion through the patient's skin. Unrestricted, those undesirable ions would be infused through the skin with the drug ions and reduce the amount of the drug delivered to the patient by a specified amount of ma-mins. By exchanging the highly conductive ionic forms of hydronium and hydroxide ions for Na + or Cl__, the competing ions' effect is reduced, allowing more medication to be infused. Using a buffer to neutralize the sodium hydroxide electrolytically generated adjacent the cathode, not only introduces more competing ions, but a buffer may chemically react with a medication being infused. As a result, the use of ion-exchange resins interferes less with the medication by merely exchanging more benign ions for the hydronium and hydroxide ions.

As shown in Figure 3, in still another embodiment of the present invention, the ion conversion material 18 may be in the form of ion-exchange membranes or sheets 50, e.g. cationic ion exchange membranes sold under the trademark, "NAFION", by DuPont of Wilmington, Delaware. Sheets of this material are generally about 0.127mm (0.005 inches) thick and may be cut to fit the particular iontophoretic bioelectrode configuration as desired. The number of these sheets equivalent in ion-exchange properties to about one- or two-cubic centimeters of ion-exchange resin, e.g. about five to about twenty sheets, may be interposed between the dispenser 20 and the metal electrode 16 to exchange deleterious electrolytically generated ions for more benign ones, e.g. $H^+$ for $Na^+$ and $OH^-$ for $Cl^-$.

Dispenser 20 is positioned between the metal electrode 16 and the patient's skin to be in electrical and fluid communication with the metal electrode 16, while being in contact with the patient's skin. The dispenser 20 may be any material capable of receiving and releasing the conductive media or medication used, e.g. wool, synthetic, natural fibers, conductive gels or any other material which has those abilities. By way of example, an absorbent pad of one-eighth inch thick orthopedic felt sold by American Felt of New York was used. The dispenser 20 should have sufficient absorption qualities to retain the desired amount of medication or electroconductive medium necessary to be applied to the patient. Impregnated conductive gels or medication releasably contained within a conductive reservoir as dispensers 20, e.g., impregnated discs or a container formed of the earlier discussed ion-exchange membranes are also contemplated.

If the ion conversion material 18 is in the form of an ion-exchange resin or membrane, retainer 22 is disposed within the interior cavity 14 to retain the ion-exchange resin therein. As shown in Figures 2 and 3, the retainer 22 may be, for example, in the form of a plastic screen having apertures or passageways sized to pass fluid or ions, but retain the ion-exchange resin, i.e. the ion conversion material 18, in close proximity to the metal electrode 16. The retainer 22 also retains the ion-exchange resin between the patient's skin and the metal electrode 16. The retainer 22 may be mounted on top of the inwardly extending annular shelf 41 to define an enclosed sub-cavity 56 for the enclosure of the ion-exchange resin. This retainer 22 may also be in the form of a water permeable, ion-exchange resin impermeable, fabric bag (not shown), within which the ion conversion material 18 is placed. This allows the retainer 22 to restrain and permit easy replacement of the ion exchange resin disposed therein. In addition, the retainer 22 may be in the form of conductive adhesive sheets such as karaya pads sold by LecTec Corporation of Eden Prairie, Minnesota.

As best shown in Figure 4, the retainer 22 may also be a conductive adhesive or electrode gel matrix 43, within which the ion-exchange resin is embedded. The electrode gel matrix 43 may be applied to the surface 58 of the metal electrode 16 or applied to the bottom surface 60 of the dispenser 20, to retain the ion-exchange resins between the metal electrode and the dispenser. In addition, the conductivity of the electrode gel may distribute the ionic charge throughout more of the ion-exchange resin, increasing its effectiveness. Examples of such electrode or adhesive gels include those gels sold under the trademarks "SPECTRA 360", "SIGNA GEL", and "TENSIVE" by Parker Laboratories of Orange, New Jersey.

For example, in one particular application of a gel matrix 43 to retain the ion-exchange resin, an anionic exchange resin XUS-40251.00 (chloride form) from Dow Chemical and conductive electrode or adhesive gels sold under the trademarks "SPECTRA 360", "SIGNA", or "TENSIVE" by Parker Laboratories, were mixed in about equal proportions, e.g. about fifty-fifty. About one cubic centimeter of each mixture was placed within a one-inch diameter bioelectrode similar in construction to the bioelectrode of Figure 4. The bioelectrode 10 was then attached to the skin of the human subject. A second iontophoretic bioelectrode 10 using cationic exchange resin was prepared in the same manner. A 2.0 ma current was applied through the bioelectrodes for varying periods of time. The results of such tests are shown in Table 2 below:

TABLE 2

| Electrode Gel | Elapsed Time Applying 2.0 ma |
|---|---|
| SPECTRA 360<br>SIGNA GEL<br>TENSIVE adhesive conductive gel | ninety minutes<br>sixty minutes<br>sixty minutes |

No iontophoretic burns beneath the attached bioelectrodes were suffered by the subject. Indeed, the elapsed times merely indicate when the current flow between the bioelectrodes was stopped and not when any burns were perceived by the patient. Thus, the use of a mixture of electrode gel and ion-exchange resin greatly increases the time a current may be applied between bioelectrodes in contact with the patient's skin without causing irritation or burn.

Furthermore, after drying out the electrode gel-resin mixtures, transforming the mixtures from the consistency of paste to a solid disc and then the resolubilizing the mixture by saturating the disc and dispenser 20 with tap water, a 2.0 ma current was applied for a period of one hour without the subject suffering from any readily discernable skin injury.

In still another example of the practice of the invention, a thin coating of the resin-gel mixture, e.g. about 0.127mm (0.005 inches) thick, was applied to the surface 60 of the dispenser 20. Application of a 2.0 ma current to the bioelectrodes for a period of one hour did not cause any readily discernable skin injury.

As best shown in Figure 5, an alternative embodiment of the improved bioelectrode 10 is a self-contained iontophoretic circuit, having a pair of electrolytic cells or electrically isolated bioelectrode portions 61 and 62, and the power source 17 for supplying an electric current across a pair of dispensers 20$'$ and 20$''$.

Referring to Figure 5, the housing 112 defines a first and second interior cavities 14$'$ and 14$''$ for receipt of the ion conversion means 18 therein. The second interior cavity 14$''$ can be concentrically formed about the first interior cavity 14$'$, separated by a cylindrical common dividing wall 70. The housing 112 also includes a third cavity 66 defined by the housing and formed at the closed back end 128 of the housing. The third cavity 66 is sized to receive the power source 17, e.g. a standard nickel-cadmium or lithium battery, to supply the necessary current between the dispenser pads 20$'$ and 20$''$. Housing 112 may be formed of any insulative material to electrically isolate the bioelectrode portions 61 and 62 from each other.

Disposed within the housing 112 are the first and second bioelectrode portions 61 and 62 in electrical communication with the anode and cathode of the power source 17. Thus, in referring to Figure 5, the reference numerals 33$'$, 33$''$, 16$'$, 16$''$, 17, 18$'$, 18$''$, 20$'$, 20$''$, 22$'$, 22$''$, 36, 41$'$ and 41$''$, denote like or corresponding elements previously described in connection with Figure 2, having substantially the same or similar characteristics.

Referring to Figure 5, this construction results in separate bioelectrode portions 61 and 62 in close proximity to, but electrically isolated from, each other by the common dividing wall 70. As a result, contacting the dispensers 20$'$ and 20$''$ with the skin will allow the iontophoretically infuse the medicine into the skin.

Referring to Figure 6, a modification of the self-contained iontophoretic circuit depicted in Figure 5 incorporates the gel matrix 43$'$ as described more fully in regards to the bioelectrode of Figure 4. Thus in referring to Figure 6, the reference numerals 112$'$, 14$'$, 14$''$, 16$'$, 16$''$, 17, 18$'$, 20$'$, 33$'$, 33$''$, 36$'$, and 43$'$, denote like or corresponding elements previously described in connection with Figures 4 and 5, having substantially the same or similar characteristics. The modifications of the bioelectrode 10 of the present embodiment are best observed with reference to Figure 6.

Again referring to Figure 6, the housing 112$'$, has a larger interior cavity 14$'$ in electrical communication with the anode of the power source 17. Indeed, the second interior cavity 14$''$ does not circumvent the first interior cavity 14$'$. Instead, the second interior cavity communicates with a top surface of the flange 36$'$. The plan defined by the top surface of the flange 36$'$ is recessed relative the plane defined by the apex of the common dividing wall 70$'$.

In some cases, the dispensers may be in both gel or absorbent felt pad form. For example, in Figure 6, there is only the dispenser 20$'$, which is in the form of an absorbent pad, electrically communicated with the anode of the power source 17. Thus, the dispenser 20 may be an absorbent pad 20$'$ or a layer of conductive gel 90, e.g., spread over the top surface of the annular flange 38$'$. The layer 90 of conductive gel retains the ion-exchange material 18$''$ between the metal electrode 16$''$ and the patient's skin. In addition, the conductive gel layer 90 may reduce the need for having the second interior cavity 14$''$ circumvent the first interior cavity 14$'$, reducing the second interior cavity's size and allowing the first interior cavity 14$'$ to be disproportionately larger than the first. This allows for a larger dispenser 20$'$ over the metal electrode 16$'$ for infusing medication. Dispenser 20$'$ is positioned to contact the patient's skin and be in electrical and fluid communication with the positive metal electrode 16.

As best shown in Figure 7, another embodiment of the bioelectrode 10 is provided for use in dentistry or dermatology. The reference numerals 16$'$, 16$''$, 18$'$, 18$''$, 20$'$, 20$''$, 22$'$, 22$''$, 41$'$, 41$''$, 70$'$, and 112$'$ in Figure 7 denote like or corresponding elements having substantially the same or similar characteristics as elements previously described having like reference numerals. The housing 112$'$ is sized to be received within a cavity 114 formed within an extension 116. In addition, the housing 112$'$ has an extension 88 at the apex of the common dividing wall 70. The extension 88 protrudes above the plane defined by a top portion 92 of the lip of the housing 112$'$. The extension 88 helps electrically isolate the two dispensers 20$'$ and 20$''$ from one another by maintaining the bioelectrode portions electrically isolated except through the patients skin and the power source 17$'$. For example, fluids, e.g., saliva or water in the patient's mouth may allow the circuit to short across the dispensers 20$'$ and 20$''$. Thus the extension 88 reduces the shorting out of the circuit across the two bioelectrodes 20' and 20'', through the fluid film.

Furthermore, as best observed in Figure 7, the orientation of the dispensers 20' and 20'' is adjusted to help maintain their contact with the skin or gums, despite the protrusion of extension 88. More specifically, the substantially horizontal planes defined by the annular shelves 41' and 41'' or the dispenser 20' or 20'' are tilted off the horizontal, e.g., the portions closest to the central longitudinal axis of the housing 112' are more proximal to the back portion 128', than the radially peripheral portions. This tilting is in the range of about 3 degrees to about 7 degrees, and preferably about 5 degrees from the horizontal and additionally serves to conform the top of the bioelectrode 10 to the desired tissue, e.g. the gums of the patient.

In still another alternative embodiment, shown in Figure 8, the ion conversion material 18' is in the form of a sacrificial electrode 100, which may be a corrosive metal which is reduced to its ionic state while concurrently oxidizing the hydronium ($H_3O^+$) ions to water or reducing the free hydroxide ($OH^-$) ions to a metal oxide complex. In the sacrificial electrode embodiment, the deleterious ions are reduced or oxidized to an oxidation-reduction state which is less irritable or injurious. As previously indicated, this also reduces the irritation or burning the iontophoretic bioelectrode 10 may cause when in contact with the patient's skin.

Referring to Figure 8, where again like reference numerals 12', 14', 16', 18', 20', 33', and 38' denote like or substantially corresponding elements previously described in connection with Figure 2, the bioelectrode 10 incorporating the sacrificial electrode 100, e.g., a disc of a corrosive metal, is interposed between the metal electrode 16 and the skin surface. The sacrificial electrode 100 would be in close proximity to, but not in direct contact with, the metal electrode 16. When the electrical current is flowing through the metal electrode 16, the electrolytically generated ions will react with the corrosive metal and be eliminated. By way of example, zinc (Zn) amphoterically converts or consumes deleterious ions through the following reactions:

$$2 H_2O + Zn(m) + 40H^- -- Zn(OH)_4{}^{2-} + H_2(g)$$

in alkaline solutions, and, by the reaction:

$$Zn(s) + 2H^+ ---- Zn^{+2} + H_2 (g)$$

in acidic solutions. Examples of sacrificial electrodes include those formed of magnesium and magnesium alloys, zinc, and various grades of aluminium. A ferrous alloy may be used in the cathode cell to react with ferrous ions to create ferrous chloride. Other metals, higher up on the galvanic series than the metals used in metal electrode 16, may be used. A pertinent galvanic series is described in the McGraw-Hill Encyclopedia of Science and Technology, Volume 3, pages 489-490, (published in 1960 by McGraw-Hill Book Company, Inc.) and is incorporated by reference herein.

The performance of the sacrificial electrode 100 may be improved by increasing the exposed surface area of the sacrificial electrode, e.g. by etching the surface area of the corrosive metal and thus causing or creating the appearance of undulations in the surface plate, or by powdering the corrosive metal.

Referring to Figure 8, the sacrificial electrode 100 may be formed of a magnesium alloy from Kaiser Aluminum of Fontana, California, designated Alloy AZ31, containing 3% aluminum and 1% zinc. The sacrificial electrode 100 may be formed into a disc, 1.9 cm (0.75 inch) in diameter and 0.76 mm (0.03 inch) thick. A separator 102, e.g., plural layers of a non-conductive, water permeable material 102, e.g. filter paper, are interposed between the metal electrode 16' and the sacrificial electrode 100. The dispenser 20' is placed over the sacrificial electrode 100 to contact the patient's skin and be in fluid communication with the skin and the metal electrode 16'. The absorption pad 20' was then saturated with tap water. In practicing the invention, initial experiments by the inventors revealed that the sacrificial electrode 100 extended the time a current may be applied through the bioelectrode 10 attached to the patient's skin without causing skin injury. See Table 3 below:

TABLE 3

|  | Current | Treatment Time until burn* | |
|---|---|---|---|
| Control | 2.0 ma | 3 minutes | none |
| Test 1 | 2.0 ma | 26 minutes | AZ31 alloy |
| Test 2 | 2.0 ma | 12 minutes | stainless steel alloy 316 |

Examination of the AZ31 alloy plate after the above test revealed high corrosion on the sacrificial electrode 100 in the area immediately over metal electrode 16. It appears the use of a sacrificial electrode 100 produced a seven-fold increase in treatment time before burn, believed primarily due to the hydroxide ions being consumed by reaction with the sacrificial electrode.

In operation, the application of the current across two adjacent dispensers 20 contacting the patient's skin will electrolytically generate H+, $H_3O^+$ ions or $OH^-$ ions, respectfully, in the particular bioelectrode 10 connected to the anode or the cathode of the power source 17. As a result, the appropriate insertion of the corresponding ion exchange resin in the respective interior cavity 14 will result in the ion-exchange and removal of the deleterious ions in solution for the less irritating ions, e.g. sodium and chloride. Alternatively, the sacrificial anode 100, by oxidation-reduction reactions, will complex with or remove the deleterious ions from adjacent the respective metal electrodes 16. As a result, the electrolytic generation of the acid or caustic components is reversed by the exchanging, reducing or oxidizing of the deleterious ions into less irritable, more benign salt constituents.

It will be appreciated from the foregoing that the present invention represents a significant advance in the field of iontophoretic bioelectrodes. In particular, the present invention provides an ion-exchange resin or a sacrificial anode between the metal electrode 16 and the medication containing dispenser 20, converting the caustic and acidic ions generated by the application of an electrical current between the bioelectrode, reducing the irritation or burning of the patient's skin. It will also be appreciated that, although presently preferred embodiments of the invention have been described by way of example, various modifications may be made without departing from the spirit and scope of the invention. Accordingly, the present invention is not to be limited except as by the appended claims.

## Claims

1. A bioelectrode for mitigating the presence of deleterious ions adjacent a patient's skin, said bioelectrode being adapted for connection to a source of electrical power, said bioelectrode comprising:
electrical contacting means for connecting said source of electrical power with a patient's skin; and
ion conversion means for removing deleterious ions, said ion conversion means being in electrical communication with said contacting means and interposed between said contacting means and the patient's skin, whereby deleterious ions generated by said electrode adjacent the patient's skin are removed from adjacent the patient's skin, thereby reducing skin irritation and injury.

2. The bioelectrode as claimed in claim 1, wherein said electrical contacting means comprises a metal electrode, said bioelectrode further comprising:
a housing, defining an interior cavity therein, said housing having an aperture adapted to communicate said interior cavity with a patient's skin, said metal electrode being disposed within said housing and, said ion conversion means being disposed within said housing and interposed between said metal electrode and the patient's skin; and
dispensing means for releasing a compound to the patient's skin, said dispensing means being in electrical communication with said ion conversion means and said metal electrode and adapted to contact the patient's skin, whereby an electrical current from said source of electrical power generates deleterious ions and said deleterious ions are scavenged by said ion conversion means, effectively removing said deleterious ions from solution, and minimizing said deleterious ions from contacting the patient's skin, thereby reducing skin injury.

3. The bioelectrode as claimed in claim 5, wherein said dispensing means includes an absorbent pad.

4. A bioelectrode as claimed in any one of the preceding claims, wherein said ion conversion means includes an ion-exchange resin in fluid communication with said contacting means and the patient's skin.

5. The bioelectrode as claimed in claim 4, and further including retaining means, said retaining means being disposed within said housing, for retaining said ion-exchange resin between said metal electrode and the patient's skin.

6. The bioelectrode as claimed in claim 5, wherein said retaining means includes an electroconductive gel for mixing with said ion-exchange resin and retaining said ion-exchange resin interposed between said metal electrode and the patient's skin.

7. A bioelectrode as claimed in any one of claims 1, 2 or 3, wherein said ion conversion means includes a sacrificial electrode, interposed between said contacting means and the patient's skin, said sacrificial electrode oxidatively-reductively converting said deleterious ions.

8. A bioelectrode as claimed in any one of claims 1, 2, 3 or 7, wherein said ion conversion means is selected from the group consisting of magnesium, zinc, or aluminum.

9. The bioelectrode as claimed in any one of claims 2, 3, 7 or 8, further including at least one sheet of an insulative material interposed between said ion conversion means and said metal electrode.

10. The bioelectrode as claimed in claim 1, wherein said electrical contacting means comprises first and second metal electrodes and further including:
a housing including a common dividing wall and defining a first, and second interior cavities, said common

9

dividing wall separating said first and second interior cavities, said ion conversion means comprising ion-exchange resin disposed within said first and second interior cavities, said first and second metal electrodes being disposed within said interior cavities, said first and second metal electrodes in electrical communication with said source of electrical power and said ion-exchange resin;

a first and second retainers disposed within said interior cavities to retain said ion-exchange resin therein; at least one absorbent pad for absorbing and releasing said medicine, said absorbent pad in electrical communication with said ion-exchange resin and in contact with the patient's skin, whereby an electrical current from said power source generates deleterious ions and said deleterious ions are reversibly bound to said ion-exchange media, effectively removing said deleterious ions from solution, to minimize contact of said deleterious ions with the patient's skin, thereby reducing skin injury.

11. The bioelectrode as claimed in claim 10, wherein said housing has a back end and defines a third interior cavity remote from said back end, and said source of electrical power is a battery received within said third interior cavity.

12. The bioelectrode as claimed in claims 10 or 11, wherein said common dividing wall has an extension extending from said common dividing wall.

13. The bioelectrode as claimed in claim 12 wherein said at least one absorbent pad is tilted relative a substantially horizontal plane.

14. A method for minimizing skin injury caused by an electrode in contact with a patient's skin, said method comprising the steps of:

placing an electrode in electrical communication with the patient's skin; and

removing deleterious ions from between said electrode and the patient's skin.

15. The method for minimizing skin injury, as set forth in claim 14, wherein said step of removing deleterious ions includes interposing an ion-exchange resin between said electrode and the patient's skin.

16. The method for minimizing skin injury as set forth in claim 14, wherein said step of removing deleterious ions includes interposing a metal which oxidative-reductively converts said deleterious ions to a more benign species.

EP 0 318 776 A1

30039

Fig.1

Fig.2

Fig.3

Fig.4

POWER SUPPLY

POWER SUPPLY

POWER SUPPLY

30039

FIG. 5

FIG. 6

FIG. 7

POWER SUPPLY

FIG. 8

POWER SUPPLY

European Patent Office

**PARTIAL EUROPEAN SEARCH REPORT** which under Rule 45 of the European Patent Convention shall be considered, for the purposes of subsequent proceedings, as the European search report

Application number

EP 88 11 9232

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO-A-87 04 936 (KEY PHARMACEUTICALS) <br><br> * Page 7, line 9 - page 11, line 28; page 16, lines 3-26; claim 3 * | 1-6,10, 11,14, 15 | A 61 N 1/30 |
| X | EP-A-0 182 520 (MEDTRONIC) <br><br> * Page 9, line 16 - page 11, line 6, page 13, lines 11-35; page 17, line 4 - page 19, line 17; figure 1 * | 1,7,8, 11,14, 16 | |
| A | EP-A-0 240 189 (THE UNIVERSITY OF UTAH RESEARCH FOUND.) <br><br> * Page 28, line 5 - page 37, line 11 * | 1,7,8, 14,16 | |
| A | US-A-3 645 260 (CINOTTI) <br><br> * Whole document * | 10-13 | TECHNICAL FIELDS SEARCHED (Int. Cl.4) <br><br> A 61 N |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-13

Claims searched incompletely:

Claims not searched: 14-16

Reason for the limitation of the search:

Method for treatment of the human or animal body by surgery or therapy (See art. 52(4) of the European Patent Convention)

| Place of search | Date of completion of the search | Examiner |
|---|---|---|
| The Hague | 16-01-1989 | SCHMIERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82